(19) Europäisches Patentamt
European Patent Office
Office européen des brevets

(11) Publication number: **0 495 548 B1**

# (12) EUROPEAN PATENT SPECIFICATION

(45) Date of publication of patent specification: **13.09.95**

(51) Int. Cl.[6]: **C07C 51/14**, C07C 67/38

(21) Application number: **92200060.9**

(22) Date of filing: **10.01.92**

## (54) Process for the carbonylation of olefin.

(30) Priority: **19.11.91 EP 91203014**
**15.01.91 GB 9100801**

(43) Date of publication of application:
**22.07.92 Bulletin 92/30**

(45) Publication of the grant of the patent:
**13.09.95 Bulletin 95/37**

(84) Designated Contracting States:
**BE DE ES FR GB IT NL**

(56) References cited:
**EP-A- 0 227 160**
**EP-A- 0 406 848**
**US-A- 3 168 553**

**PATENT ABSTRACTS OF JAPAN, vol. 5, no. 111 (C-63)(783), 18th July 1981; & JP-A-56 49 333**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**
Inventor: **Kragtwijk, Eric**
**Badhuisweg 3**
**NL-1031 CM Amsterdam (NL)**

EP 0 495 548 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the carbonylation of olefins by reaction with carbon monoxide in the presence of a catalyst system.

The invention in particular concerns the preparation of alkylpropionates which are commercially used as solvents and in flavouring compositions and perfumes.

Furthermore the invention relates to certain catalyst systems, suitable to be used in the carbonylation process.

Various processes for the carbonylation of olefins are already known. In order to improve the conversion of the olefin, the selectivity towards the desired product, or both, many modifications have been tried out which, although effective in one respect, are usually unfavourable in another aspect.

For example, in EP 279477 a continuous process for the carbonylation of an alkene is disclosed using a catalyst system comprising palladium or a palladium compound, a ligand, in particular triphenylphosphine and an acid, wherein the ligand and the acid are added either continuously or intermittently and whereby catalyst removed from the reaction vessel is recycled. Whereas in this process high selectivities with respect to the desired methylpropionate are obtainable, the consumption of catalyst components per kg methylpropionate is relatively high notwithstanding the catalyst recycle step.

In other processes, e.g. the process described in US 3,168,553 catalyst systems comprising as metal component rhodium, ruthenium, iridium, or, in particular cobalt are used. With the aid of these catalyst systems, relatively low yields of the desired ester are obtained and moreover these processes are usually unattractive in view of the required high pressures.

According to another process, described in EP 411721, alkylpropionates can be produced continuously by reacting an alkanol in the liquid phase with ethene and carbon monoxide in the presence of a carbonylation catalyst and removing alkylpropionate from the reactor vessel in a stream of vapour. The catalyst is based on a palladium compound, a large excess of a phosphorus-, arsenic-, or antimony-containing ligand, in particular triarylphosphine and a protonic acid, e.g. sulphuric acid.

In the carbonylation process described in EP 55875, an olefin is reacted with carbon monoxide in the presence of water, an alcohol or a carboxylic acid. As catalyst a system is used comprising a palladium component or a cobalt-containing catalyst together with the palladium component, a tri-organophosphine promoter, containing at least one aliphatic carbon atom bonded to phosphorus. The molar ratio between the organophosphine and palladium is less than 10:1. In practice still relatively high molar ratios, e.g. of 6:1 or 7:1 are used.

In the non-prepublished UK patent application (No. 9100801.1) a carbonylation process is described whereby use is made of a catalyst system comprising a source of palladium cations, a source of bidentate phosphine wherein the two phosphorus atoms are bonded to aliphatic groups, and a source of anions derived from a strong acid. The process is carried out in the presence of a hydride source and the carbonylation products are mainly aldehydes and/or ketones.

EP-A-406848 discloses a catalyst system which is a complex of palladium with a chelating phosphine bidentate ligand and is used to directly carbonylate arylchlorides to aldehydes through the formylation reaction (reaction of the arylchloride with carbon monoxide and a formate salt). The preferred phosphine ligand is 1,3-bis(diisopropylphosphino)-propane (dippp) and the catalyst formula $(dippp)_2Pd$.

It has now been found that in the carbonylation of olefins unexpected advantages are obtained, if the reaction is carried out in the presence of a catalyst system based on a ligand selected from a special group of bidentates. In many cases particularly high reaction rates are thus attained.

The invention can be defined as relating to a process for the carbonylation of olefins in which process an olefin is reacted with carbon monoxide in the presence of an alcohol or water and of a catalyst system, obtainable by combining:

(a) a metal of Group VIII or a compound thereof and

(b) a bidentate phosphine, arsine and/or stibine derivative, wherein as bidentate (b) a compound is selected having the general formula

$$R^1R^2\text{-}M^1\text{-}R\text{-}M^2\text{-}R^3R^4 \qquad I$$

wherein $M^1$ and $M^2$ are independently P, As or Sb, R is a divalent organic bridging group with at least 2 carbon atoms in the bridge, and $R^1$-$R^4$ represent the same or different optionally substituted tertiary alkyl groups.

In the bidentate of formula I, $M^1$ and $M^2$ are preferably the same and in particular they both represent phosphorus atoms.

The bridging group R usually contains from 2 to 5 carbon atoms which may be substituted by one or more substituents such as alkyl groups, e.g. of 1 to 4 carbon atoms, alkoxy groups in which the alkyl group comprises from 1 to 3 carbon atoms, dialkylamino groups in which the alkyl groups independently comprise 1 to 3 carbon atoms, or halogen atoms such as bromine and chlorine atoms.

In the bridging group the carbon chain may be interrupted by one or more heteroatoms such as oxygen or sulphur atoms, or by silano or dialkylsilicon groups in which the alkyl groups independently comprise from 1 to 4 carbon atoms. Preferably the bridging group does not contain terminal heteroatoms.

Examples of suitable tertiary alkyl groups represented by $R^1$ to $R^4$ are tertiary butyl, 2-(2-methyl)butyl, 2-(2-ethyl)butyl, 2-(2-methyl)pentyl and 2-(2-ethyl)pentyl groups. In the present specification the alkyl groups represented by $R^1$ to $R^4$ include cyclic structures such as a 1-norbornyl or 1-norbornadienyl group. Preferably the groups $R^1$ to $R^4$ represent the same tertiary alkyl groups.

A particularly preferred bidentate is 1,3-bis(di-tertiary-butylphosphino)propane.

Among the metals of Group VIII, cobalt, nickel, palladium, rhodium and platinum may be mentioned. Of these, palladium is in particular preferred. As source of Group VIII metal, hereinafter further exemplified as source of palladium, metallic palladium or, preferably, a palladium compound may be used, in particular a palladium salt. Examples of suitable salts are salts of nitric acid, sulphuric acid, sulphonic acids such as methane sulphonic acid, chlorosulphonic acid, trifluoro methane sulphonic acid, a toluene sulphonic acid, e.g. p-toluene sulphonic acid, t-butyl sulphonic acid and sulphonated ion exchange resins.

Furthermore palladium salts of alkanoic acids may be used, in particular alkanoic acids with up to 12 carbon atoms, for example acetic acid, propionic acid or trifluoroacetic acid.

Preferred catalyst systems are based on palladium salts of strong acids, i.e. acids having a pKa value of at most 2.5, or a derivative thereof. Examples are trifluoroacetic acid and p-toluenesulphonic acids in combination with bidentates wherein $R^1$ to $R^4$ represent tertiary alkyl groups, in particular tertiary butyl groups. It is believed that such catalyst systems comprise palladium in cationic form.

The amount of acid may exceed the stoichiometric amount required for the palladium salt, if so desired.

Since halide ions can be corrosive, the source of palladium in the catalyst systems of the invention is preferably not a halide or a compound generating halide ions.

Conveniently the catalyst system of the invention is obtained by combining in a separate step, preceding the carbonylation reaction, the source of palladium and the bidentate of formula I. Suitably the palladium compound, as exemplified hereinbefore, is dissolved in a suitable solvent, and subsequently admixed with the bidentate. The molar ratio between the bidentate (b) and the palladium source (a) is preferably in the range of 1:1 to 5:1 and, more preferably, in the range of 1:1 to 3:1. The possibility of applying these low molar ratios is advantageous, as it avoids the use of an excess of bidentate (b) and hence minimizes the consumption of these usually expensive compounds.

The amount of catalyst used in the process is not critical. Good results are obtained when the amount of Group VIII metal is in the range of $10^{-7}$ to $10^{-1}$ gat per mole of olefinic double bond to be carbonylated. Preferably this amount is in the range of $10^{-5}$ to $5.10^{-2}$ gat per mole.

A wide range of olefins may be used in the process of the invention. Suitable olefins include those having 2 to 30 carbon atoms, in particular olefins with 2 to 12 carbon atoms per molecule. Preferably lower olefins are applied having from 2 to 6 carbon atoms, e.g. ethene, propene, butenes, and pentenes. Ethene and propene are in particular preferred.

If desired olefins having more than one ethylenically unsaturated double bond may be used, in particular those wherein the double bonds are non conjugated, such as 1,5-hexadiene. Mixtures of olefins may be used, but in general product recovery is easiest if a single end product is aimed at.

Olefins wherein one or more hydrogen atoms have been replaced by inert substituents may also be used. Examples of inert substituents are halogen atoms and cyano, ester, alkoxy, aryl, hydroxy, and carboxy groups. Olefins thus substituted are for example styrene and alkylesters of unsaturated carboxylic acids, such as methylacrylate.

If the carbonylation process is carried out in the presence of water, the product obtained will be a carboxylic acid. The acid may be converted into other products such as amides or esters.

In the process according to the invention, esters may be obtained directly, if the carbonylation is carried out in the presence of an alcohol.

Suitable alcohols include aliphatic mono alcohols, in particular those having from 1-5 carbon atoms per molecule such as methanol, butanol, isopropanol, and dihydric alcohols such as ethylene glycol and 1,3-propane diol.

Methanol is in particular preferred.

The amount of alcohol is not critical. Generally, amounts are used in excess of the amount of olefin to be carbonylated. Thus the alcohol may serve as reaction solvent as well, although, if desired, separate

solvents may also be used.

In addition to a reaction solvent, if any, the reaction medium may contain one or more promoters.

Suitable promoters include organic oxidant promoters such as quinones and nitrocompounds. Moreover drying agents may be present such as trimethyl ortho formate.

The amount of promoter is not critical, but is conveniently related to the amount of catalyst used in the process. Accordingly the amount of promoter is usually in the range of 0.001 to 10 moles per mole of olefinic double bond to be carbonylated, in particular in the range of 0.01 to 5 moles per mole.

The carbonylation reaction according to the invention is carried out at moderate temperatures and pressures. Suitable reaction temperatures are in the range of 50-250 °C, preferably in the range of 75-150 °C. Reaction temperatures outside these ranges may be applied, but generally do not offer special advantages.

The reaction pressure is usually at least atmospheric. Suitable pressures are in the range of 1 to 100 bar, preferably in the range of 5 to 50 bar.

The process may be carried out in batch operation or continuously. In embodiments relating to continuous operation of the process, products are conveniently stripped from the reaction mixture with the aid of a gas, usually feed gas, and subsequently recovered. The stripping gas is suitably returned to the reaction zone.

The carbon monoxide required for the reaction may be supplied in substantially pure form, or contaminated with in general minor amounts of inert compounds such as nitrogen, hydrogen and the like. The presence of sulphur containing contaminants such as COS or some metal compounds e.g. metal carbonyl compounds, should be avoided.

Hydrogen or a hydrogen containing gas may be present as a diluent for the carbon monoxide and other gaseous reactants. The pressure at which the hydrogen is supplied may vary, but is usually not more than that of the CO partial pressure.

The invention may be illustrated by the following Examples: Unless otherwise stated, all experiments were carried out in the following manner.

A 300 ml magnet-driven autoclave of Hastelloy C (Trade Mark) was charged with alkanol and further liquid and solid components of the reaction mixture as stated for each specific example. The catalyst components were dissolved in the respective solvent (usually the alkanol) under nitrogen atmosphere in a glove box and introduced in the nitrogen blanketed autoclave. The autoclave was closed, evacuated and subsequently pressurized with carbon monoxide and any other stated gaseous component. The autoclave was then heated to the indicated temperature and maintained at that temperature, usually for a period between 0.25 and 5 hours.

Finally the contents of the autoclave were analysed by gas-liquid chromatography.

Examples I to XIII

In Table I below the following data are indicated:
the catalyst components present in the reaction mixture (in mmoles);
the alkanol and any further liquid (diluent) (in ml);
the olefin used and the pressures of the olefin, carbon monoxide and any other gas (in bar, unless otherwise mentioned);
the reaction temperature (in °C).

In Table II for each example the reaction rate (in moles of ester produced per gat of palladium and per hour) and the selectivity with respect to the desired product (in %) are shown.

Table I

| Example No | Catalyst components (mmol) | Alkanol, etc. (ml) | Pressure (bar) olefin | CO | other | Temperature (°C) |
|---|---|---|---|---|---|---|
| I | 0.1 palladium acetate<br>0.3 1,3-bis(di-tert. butylphosphino)-propane<br>0.25 tert.butyl sulphonic acid | 40 butanol-1 | ethene 20 | 40 | $H_2$ 5 | 110 |
| II | 0.1 palladium acetate<br>0.3 1,3-bis(di-tert. butylphosphino)-propane<br>0.25 methylsulphonic acid | 40 butanol-1<br>40 methylpro-pionate | ethene 20 | 40 | $H_2$ 5 | 110 |
| III | 0.1 palladiumacetate<br>0.3 1,3-bis(di-tert. butylphosphino)-propane<br>0.25 tert.butyl sulphonic acid | 20 methanol<br>30 methylpro-pionate | ethene 20 | 40 | | 100 |

Table I (Cont.)

| Example No | Catalyst components (mmol) | Alkanol, etc. (ml) | Pressure (bar) olefin | Pressure (bar) CO | Pressure (bar) other | Temperature (°C) |
|---|---|---|---|---|---|---|
| IV | 0.1 palladiumacetate<br>0.3 1,3-bis(di-tert. butylphosphino)-propane<br>0.25 methylsulphonic acid | 20 methanol<br>30 methylpro-pionate | ethene 20 | 40 | $H_2$ 5 | 100 |
| V | 0.1 palladiumacetate<br>0.3 1,3-bis(di-tert. butylphosphino)-propane<br>0.25 methylsulphonic acid | 20 methanol<br>40 diglyme (dimethyl ether of di-ethyleneglycol) | propene 20 ml | 30 | $H_2$ 30 | 90 |
| VI | 0.1 palladiumacetate<br>0.3 1,3-bis(di-tert. butylphosphino)-propane<br>0.25 tert.butylsulphonic acid | 50 methanol<br>5 trimethyl orthoformate | octene-1 (20 ml) | 40 | | 65 |

Table I (Cont.)

| Example No | Catalyst components (mmol) | Alkanol, etc. (ml) | Pressure (bar) olefin | CO | other | Temperature (°C) |
|---|---|---|---|---|---|---|
| VII | 0.1 palladiumacetate<br>0.3 1,3-bis(di-tert. butylphosphino-propane<br>0.25 tert.butylsulphonic acid | 30 methanol | styrene (20 ml) | 40 | | 75 |
| VIII | 0.1 palladiumacetate<br>0.3 1,3-bis(di-tert. butylphosphino-propane<br>0.25 tert.butylsulphonic acid | 10 water<br>45 N-methyl-pyrrolidone | ethene 20 | 40 | | 90 |
| IX | 0.25 palladiumacetate<br>0.6 1,3-bis(di-tert. butylphosphino)-propane<br>0.75 tert.butylsulphonic acid | 40 methanol<br>5 trimethyl ortho-formate | methylacrylate (20 ml) | 40 | | 90 |

Table I (Cont.)

| Example No | Catalyst components (mmol) | Alkanol, etc. (ml) | Pressure (bar) olefin | CO | other | Temperature (°C) |
|---|---|---|---|---|---|---|
| X | 0.25 palladiumacetate<br>0.6 1,3-bis(di-tert. butylphosphino)-propane<br>0.6 tert.butylsulphonic acid | 40 methanol | methylacrylate (20 ml) | 40 | $H_2$ 5 | 90 |
| XI | 0.25 palladiumacetate<br>0.6 1,3-bis(di-tert. butylphosphino)-propane<br>0.6 tert.butylsulphonic acid | 50 methanol<br>5 trimethyl ortho-formate | acrylamide (15 g) | 40 | | 115 |
| XII | 0.25 palladiumacetate<br>0.6 1,3-bis(di-tert. butylphosphino)-propane<br>0.5 tert.butylsulphonic acid | 20 methanol<br>40 diglyme | vinylacetate (20 ml) | 40 | | 75 |

Table I (Cont.)

| Example No | Catalyst components (mmol) | Alkanol, etc. (ml) | Pressure (bar) olefin | CO | other | Temperature (°C) |
|---|---|---|---|---|---|---|
| XIII | 0.25 palladiumacetate<br>0.6 1,3-bis(di-tert. butylphosphino)-propane<br>1 trifluoromethyl-sulphonic acid | 50 methanol<br>5 trimethyl ortho-formate | cyanoethene (20 ml) | 40 | | 60 |
| A (comparative) | 0.1 palladiumacetate<br>3 triphenylphosphine<br>2 methylsulphonic acid | 20 methanol<br>30 methylpropionate | ethene 20 | 40 | | 100 |
| B (comparative) | 0.1 palladiumacetate<br>0.3 triphenylphosphine<br>0.25 methylsulphonic acid | 20 methanol<br>30 methylpropionate | ethene 20 | 40 | | 100 |

Table II

| Example No | Reaction rate (mol/gat.h) | Product selectivity (%) | |
|---|---|---|---|
| I | 11000 | butylpropionate | >98 |
| II | 10000 | butylpropionate | >98 |
| III | 5000 | methylpropionate | >98 |
| IV | 13000 | methylpropionate | >98 |
| V | 2000 | methylbutanoate | 86 |
| | | methyl-(2-methylpropionate) | 14 |
| VI | 100 | methylester of n-octanecarboxylic acid | 86 |
| | | methylester of branched octanecarboxylic acid | 14 |
| VII | 100 | methylester of 1-phenylpropionic acid | 88 |
| | | methylester of 2-phenylpropionic acid | 12 |
| VIII | 1000 | propionic acid | >98 |
| IX | 1500 | dimethylsuccinate | 98 |
| X | 3000 | dimethylsuccinate | 98 |

Table II (Cont.)

| Example No | Reaction rate (mol/gat.h) | Product selectivity (%) | |
|---|---|---|---|
| XI | 400 | methylester of mono amidosuccinic acid | 75 |
| | | succinimide | 20 |
| XII | 200 | 1-acetoxy methyl propionate | 40 |
| | | 2-acetoxy methyl propionate | 60 |
| XIII | 30 | 2-cyano methyl propionate | 95 |
| A (Comparative) | 400 | methylpropionate | 98 |
| B | <10 | methylpropionate (trace) | |

In the Tables two further examples are included for comparison only (Examples A and B).

Comparing the results of Example A with those of Example III, it will be clear that if instead of the bidentate catalyst component according to the invention, triphenylphosphine is used, large amounts thereof are required in order to achieve an acceptable reaction rate (Example A). If the phosphine is applied in an amount comparable to that used in Example III, the reaction rate is very low and only traces of methylpropionate are formed (Example B).

Another comparative experiment was carried out whereby the autoclave was charged with 10 ml methanol, 30 ml methylpropionate, 0.25 mmol palladiumacetate, 0.6 mmol 1,3-bis(di-sec.butylphosphino)-propane (instead of the bidentate of the invention) and 1 mmol tert.butylsulphonic acid. After flushing with carbon monoxide the reactor was pressurized with 20 bar ethene and 42 bar carbonmonoxide. The reaction temperature was maintained at 110 °C. After 3 hours the reaction was discontinued. 6 Gram of polymeric material (polyketones) had been formed. The selectivity with respect to methylpropionate was less than 30% (comparative Example C).

## Claims

1. A process for the carbonylation of olefins in which process an olefin is reacted with carbon monoxide in the presence of an alcohol or water and of a catalyst system, obtainable by combining:
   (a) a metal of Group VIII or a compound thereof and
   (b) a bidentate phosphine, arsine and/or stibine derivative, wherein as bidentate (b) a compound is selected having the general formula

   $R^1R^2\text{-}M^1\text{-}R\text{-}M^2\text{-}R^3R^4$ I

   wherein $M^1$ and $M^2$ are independently P, As or Sb, R is a divalent organic bridging group with at least 2 carbon atoms in the bridge, and $R^1$-$R^4$ represent the same or different optionally substituted

tertiary alkyl groups.

**2.** A process as claimed in claim 1, characterized in that the bidentate of formula I is a bisphosphine wherein $R^1$-$R^4$ represent the same tertiary alkyl groups.

**3.** A process as claimed in claim 1 or 2, characterized in that $R^1$-$R^4$ represent tertiaty butyl groups.

**4.** A process as claimed in one or more of claims 1-3, characterized in that the bidentate (b) is 1,3-bis(di-tert.butylphosphino)propane.

**5.** A process as claimed in one or more of claims 1-4, characterized in that the Group VIII metal is palladium.

**6.** A process as claimed in one or more of claims 1-5, characterized in that the molar ratio between (b) and (a) is in the range of 1:1 to 5:1.

**7.** A process as claimed in one or more of claims 1-6, characterized in that in the carbonylation reaction an olefin is used having from 2 to 30 carbon atoms being unsubstituted or substituted by one or more of the same or different substituents selected from halogen atoms, cyano, ester, alkoxy, aryl, hydroxy and carboxy groups.

**8.** A process as claimed in claim 7, characterized in that the olefin is an unsubstituted alkene having from 2 to 6 carbon atoms in particular ethene.

**9.** A process as claimed in one or more of claims 1-7, characterized in that the olefin is an ester of an unsaturated carboxylic acid.

**10.** A process as claimed in one or more of claims 1-9, characterized in that in the carbonylation reaction an aliphatic alcohol having from 1-5 carbon atoms is used.

**11.** A process as claimed in claim 10, characterized in that the alcohol is methanol.

**12.** A catalyst system comprising:
(a) a source of palladium cations
(b) a source of bidentate phosphine having the general formula I wherein $M^1$ and $M^2$ represent phosphorus atoms, R is a divalent organic bridging group with 3 carbon atoms and $R^1$-$R^4$ are tertiary alkyl groups.

**13.** A catalyst system as claimed in claim 12 wherein $R^1$-$R^4$ are tertiary butyl groups.

## Patentansprüche

**1.** Verfahren zur Carbonylierung von Olefinen, bei dem ein Olefin in Gegenwart eines Alkohols oder von Wasser und in Gegenwart eines Katalysatorsystems, erhältlich durch Kombination von
(a) einem Metall der Gruppe VIII oder einer seiner Verbindungen mit
(b) einem zweizähnigen Phosphin-, Arsin- und/oder Stibinderivat, wobei man als zweizähnigen Liganden (b) eine Verbindung der allgemeinen Formel

$$R^1R^2\text{-}M^1\text{-}R\text{-}M^2\text{-}R^3R^4 \qquad I$$

auswählt, in der $M^1$ und $M^2$ unabhängig P, As oder Sb bedeuten, R eine bivalente organische Brückengruppe mit mindestens 2 Kohlenstoffatomen in der Brücke bedeutet und $R^1$-$R^4$ für gleiche oder verschiedene, gegebenenfalls substituierte tertiäre Alkylgruppen stehen,
mit Kohlenmonoxid umgesetzt wird.

**2.** Verfahren nach Anspruch 1, dadurch gekennzeichnet, daß es sich bei dem zweizähnigen Liganden der Formel I um ein Bisphosphin handelt, in dem $R^1$-$R^4$ für die gleichen tertiären Alkylgruppen stehen.

3. Verfahren nach Anspruch 1 oder 2, dadurch gekennzeichnet, daß $R^1$-$R^4$ für tertiäre Butylgruppen stehen.

4. Verfahren nach einem oder mehreren der Ansprüche 1-3, dadurch gekennzeichnet, daß es sich bei dem zweizähnigen Liganden (b) um 1,3-Bis(di-tert.butylphosphino)propan handelt.

5. Verfahren nach einem oder mehreren der Ansprüche 1-4, dadurch gekennzeichnet, daß es sich bei dem Metall der Gruppe VIII um Palladium handelt.

6. Verfahren nach einem oder mehreren der Ansprüche 1-5, dadurch gekennzeichnet, daß das molare Verhältnis zwischen (b) und (a) im Bereich von 1:1 bis 5:1 liegt.

7. Verfahren nach einem oder mehreren der Ansprüche 1-6, dadurch gekennzeichnet, daß man für die Carbonylierungsreaktion ein Olefin mit 2 bis 30 Kohlenstoffatomen verwendet, die unsubstituiert oder durch einen oder mehrere gleiche oder verschiedene, aus Halogenatomen, Cyano-, Ester-, Alkoxy-, Aryl-, Hydroxyl- und Carboxylgruppen ausgewählte Substituenten substituiert sind.

8. Verfahren nach Anspruch 7, dadurch gekennzeichnet, daß es sich bei dem Olefin um ein unsubstituiertes Alken mit 2 bis 6 Kohlenstoffatomen und insbesondere um Ethen handelt.

9. Verfahren nach einem oder mehreren der Ansprüche 1-7, dadurch gekennzeichnet, daß es sich bei dem Olefin um den Ester einer ungesättigten Carbonsäure handelt.

10. Verfahren nach einem oder mehreren der Ansprüche 1-9, dadurch gekennzeichnet, daß man für die Carbonylierungsreaktion einen aliphatischen Alkohol mit 1-5 Kohlenstoffatomen verwendet.

11. Verfahren nach Anspruch 10, dadurch gekennzeichnet, daß es sich bei dem Alkohol um Methanol handelt.

12. Katalysatorsystem, enthaltend:
(a) eine Quelle für Palladiumkationen
(b) eine Quelle für zweizähniges Phosphin der allgemeinen Formel I, in der $M^1$ und $M^2$ für Phosphoratome stehen, R eine bivalente organische Brückengruppe mit 3 Kohlenstoffatomen bedeutet und $R^1$-$R^4$ tertiäre Alkylgruppen bedeuten.

13. Katalysatorsystem nach Anspruch 12, in dem $R^1$-$R^4$ tertiäre Butylgruppen bedeuten.

## Revendications

1. Un procédé pour la carbonylation d'oléfines dans lequel une oléfine est mise à réagir avec le monoxyde de carbone en présence d'un alcool ou d'eau et d'un système catalytique, pouvant être obtenu en combinant :
(a) un métal du groupe VIII ou un composé d'un tel métal et
(b) un dérivé bidenté de phosphine, d'arsine ou de stibine, où comme dérivé bidenté (b) on utilise un composé ayant la formule générale

$$R^1R^2\text{-}M^1\text{-}R\text{-}M^2\text{-}R^3R^4$$

dans laquelle $M^1$ et $M^2$ sont indépendamment P, As ou Sb, R est un groupe organique divalent formant pont ayant au moins 2 atomes de carbone dans le pont, et $R^1$-$R^4$ représentent des groupes alcoyle tertiaire éventuellement substitués identiques ou différents.

2. Un procédé selon la revendication 1, caractérisé en ce que le constituant bidenté de formule I est une bisphosphine dans laquelle $R^1$-$R^4$ représentent les mêmes groupes alcoyle tertiaire.

3. Un procédé selon la revendication 1 ou 2, caractérisé en ce que $R^1$-$R^4$ représentent des groupes butyle tertiaire.

4. Un procédé selon une ou plusieurs des revendications 1-3, caractérisé en ce que le constituant bidenté (b) est le 1,3-bis(di-tert-butylphosphino)propane.

5. Un procédé selon une ou plusieurs des revendications 1-4, caractérisé en ce que le métal du groupe VIII est le palladium.

6. Un procédé selon une ou plusieurs des revendications 1-5, caractérisé en ce que le rapport molaire entre (b) et (a) est compris entre 1:1 et 5:1.

7. Un procédé selon une ou plusieurs des revendications 1-6, caractérisé en ce que dans la réaction de carbonylation on utilise une oléfine ayant de 2 à 30 atomes de carbone qui est non-substituée ou est substituée par un ou plusieurs substituants identiques ou différents choisis parmi des atomes d'halogènes et des groupes cyano, ester, alcoxy, aryle, hydroxy et carboxy.

8. Un procédé selon la revendication 7, caractérisé en ce que l'oléfne est un alcène non-substitué ayant de 2 à 6 atomes de carbone, en particulier l'éthène.

9. Un procédé selon une ou plusieurs des revendications 1-7, caractérisé en ce que l'oléfine est un ester d un acide carboxylique insaturé.

10. Un procédé selon une ou plusieurs des revendications 1-9, caractérisé en ce que dans la réaction de carbonylation on utilise un alcool aliphatique ayant de 1 à 5 atomes de carbone.

11. Un procédé selon la revendication 10, caractérisé en ce que l'alcool est le méthanol.

12. Un système catalytique comprenant :
(a) une source de cations de palladium
(b) une source de phosphine bidentée ayant la formule générale I dans laquelle $M^1$ et $M^2$ représentent des atomes de phosphore, R est un groupe organique divalent formant pont de 3 atomes de carbone et $R^1$-$R^4$ sont des groupes alcoyle tertiaire.

13. Un système catalytique selon la revendication 12 dans lequel $R^1$-$R^4$ sont des groupes butyle tertiaire.